Europäisches Patentamt

European Patent Office

Office européen des brevets

(19)

(11) Veröffentlichungsnummer: **0 449 178 A2**

# (12) EUROPÄISCHE PATENTANMELDUNG

(21) Anmeldenummer: 91104674.6

(22) Anmeldetag: 25.03.91

(51) Int. Cl.⁵: **C12N 15/66**, C12N 9/22, C12P 19/34

(30) Priorität: 26.03.90 DE 4009663

(43) Veröffentlichungstag der Anmeldung:
02.10.91 Patentblatt 91/40

(84) Benannte Vertragsstaaten:
AT BE CH DE DK ES FR GB GR IT LI LU NL SE

(71) Anmelder: BOEHRINGER MANNHEIM GMBH
Sandhofer Strasse 112-132
W-6800 Mannheim-Waldhof(DE)

(72) Erfinder: Laue, Frank
Seestrasse 16
W-8121 Pähl(DE)
Erfinder: Ankenbauer, Waltraud, Dr. rer. nat.
Flohbühlweg 2
W-8122 Penzberg(DE)
Erfinder: Schmitz, Gudrun, Dr. rer. nat.
Wettersteinstrasse 3
W-8139 Bernried(DE)

(74) Vertreter: Huber, Bernhard, Dipl.-Chem. et al
Möhlstrasse 22 Postfach 860 820
W-8000 München 86(DE)

(54) Verfahren zur Verminderung von Sternaktivitäten.

(57) Zur Verminderung von unspezifischen Sternaktivitäten bei der spezifischen Spaltung von Desoxyribonukleinsäuren durch Inkubation mit einer Restriktionsendonuklease in einem geeigneten Puffer setzt man dem Inkubationsansatz ein Antibiotikum zu, welches an oder nahe der Sternsequenzen des Enzyms, jedoch nicht innerhalb der spezifischen Erkennungssequenz der Restriktionsendonuklease an die DNA bindet.

EP 0 449 178 A2

Die Erfindung betrifft ein Verfahren zur Verminderung von unspezifischen Sternaktivitäten bei der spezifischen Spaltung von Desoxyribonukleinsäuren durch Inkubation mit einer Restriktionsendonuklease in einem geeigneten Puffer.

Seit nunmehr beinahe 20 Jahren werden Restriktionsenzyme für die Spaltung von Desoxyribonukleinsäuren verwendet. Die sogenannten Typ II Restriktionsendonukleasen erkennen hierbei spezifische Sequenzen doppelsträngiger DNA und spalten die DNA innerhalb dieser Erkennungssequenzen. Bei vielen Restriktionsendonukleasen ist jedoch unter bestimmten Inkubationsbedingungen die Spezifität vermindert, sie zeigen eine sogenannte "Sternaktivität". Dabei werden Sequenzen, im folgenden Sternsequenzen genannt, gespalten, die der Erkennungssequenz ähnlich, aber nicht mit ihr identisch sind (B. Polisky, P. Greene, D.E. Garfin, B.J. McCarthy, H.M. Goodman & H.W. Boyer, Proc. Natl. Acad. Sci. USA 22 (1975), 3310). Solche Sternaktivitäten sind für viele Enzyme wie z.B. EcoRI (EcoRI*) festgestellt worden. Da die Sternaktivität eine dem Enzym inhärente Eigenschaft ist, kann sie auch nicht durch eine weitere Aufreinigung der Enzympräparation vermieden werden. Sternaktivität wird im allgemeinen insbesondere dann beobachtet, wenn hohe Endonuklease-Konzentrationen, lange Inkubationszeiten, niedrige Ionenstärke, hoher pH-Wert, niedrige Temperaturen und organische Lösungsmittel, wie z.B. DMSO oder Glycerin verwendet werden, die die doppelsträngige DNA-Struktur destabilisieren.

Es wurde zwar versucht, durch Erhöhung der Ionenstärke die Sternaktivität zu unterdrücken, allerdings ist dies häufig mit einem Verlust an Enzymaktivität verbunden. Eine weitere Möglichkeit zur Unterdrückung der Sternaktivität ist der Zusatz von Spermidin zum Inkubationsansatz (Pingoud, A. (1985) Eur.J.Biochem. 147, 105-109). Dabei wird jedoch auch die spezifische Aktivität vermindert. Bei einigen Enzymen wird selbst unter diesen Bedingungen noch Sternaktivität beobachtet.

Aufgabe der vorliegenden Erfindung ist es daher, ein Verfahren bereitzustellen, das es erlaubt, ohne eine Verminderung der spezifischen Enzymaktivität die unspezifischen Sternaktivitäten von Restriktionsendonukleasen weitgehend zu verringern und das universell unter üblichen Inkubationsbedingungen anwendbar ist.

Gegenstand der vorliegenden Erfindung ist daher ein Verfahren zur Verminderung von unspezifischen Sternaktivitäten bei der spezifischen Spaltung von Desoxyribonukleinsäuren durch Inkubation mit einer Restriktionsendonuklease in einem geeigneten Puffer, das dadurch gekennzeichnet ist, daß man dem Inkubationsansatz ein Antibiotikum zusetzt, welches an oder nahe der Sternsequenzen des Enzyms, jedoch nicht innerhalb der spezifischen Erkennungssequenz der Restriktionsendonuklease an die DNA bindet.

Das erfindungsgemäße Verfahren wird unter Standardbedingungen für das jeweils verwendete Restriktionsenzym durchgeführt, nämlich vorzugsweise in einem pH-Bereich von 7,5 bis 8,0, bei Tris-Acetat- oder Tris-HCl-Konzentrationen von 10 bis 50 mmol/l, Magnesiumacetat oder Magnesiumchlorid in Konzentrationen von 5 bis 10 mmol/l, Kaliumacetat von 66 mmol/l oder Natriumchlorid in Konzentrationen zwischen 0 und 100 mmol/l. Als Reduktionsmittel können DTE (1,4-Dithioerythrit 1 mmol/l), DTT (1,4-Dithiothreit 0,5 mmol/l) oder 2-Mercaptoethanol (1 mmol/l) eingesetzt werden. Besonders bevorzugte Bedingungen sind in der Broschüre der Firma Boehringer Mannheim "Biochemicals for Molecular Biology" (1988), Seiten 176/177 und in ähnlicher Form in Maniatis et al., (1982) Molecular Cloning, A Laboratory Manual, Cold Spring Harbor Laboratory sowie O'Farrell et al. (1980) Mol. Gen. Genet. 179, 421 beschrieben. Dort ist für eine Vielzahl von Restriktionsenzymen eine jeweils besonders geeignete Pufferzusammensetzung angegeben.

Durch den Zusatz des DNA-bindenden Antibiotikums werden die Sternaktivitäten deutlich vermindert, wenn nicht gar gänzlich beseitigt. Dies erfolgt durch Bindung des Antibiotikums an die oder nahe der Sternsequenzen des Restriktionsenzyms auf der DNA und damit Schutz der Sequenzen vor Spaltung. Es ist jedoch wesentlich für das erfindungsgemäße Verfahren, daß nicht auch eine Bindung der spezifischen Erkennungssequenz des Restriktionsenzyms durch das Antibiotikum erfolgt, da hierdurch die spezifische Aktivität ebenfalls stark beeinträchtigt würde.

Um für ein jeweiliges Restriktionsenzym ein geeignetes, die Sternaktivitäten verringerndes Antibiotikum zu finden, das jedoch die spezifische Aktivität des Enzyms nicht beeinträchtigt, sind erfindungsgemäß zwei Arten von Orientierungsversuchen durchzuführen. Erstens muß sichergestellt werden, daß das verwendete Antibiotikum nicht an die spezifische Erkennungssequenz der Restriktionsendonuklease bindet. Hierfür wird vorzugsweise ein sogenannter "footprint assay" (R. J. White und D.R. Phillips (1989) Biochemistry 28, 6259-6269; W.S. Dynan und R. Tjian (1983), Cell 35, 79-87) durchgeführt. Nach dieser Methode wird das DNA-bindende Antibiotikum an ein DNA-Fragment gebunden, das mindestens einmal die spezifische Erkennungssequenz der Restriktionsendonuklease enthält, und die DNA dann so mit DNAse abgebaut, daß maximal ein Schnitt pro DNA-Fragment entsteht. Bei der Auftrennung der Spaltprodukte im denaturierenden Polyacrylamidgel entsteht ein leiterartiges Muster, in dem sich dort Lücken befinden, wo das Bindeprotein die DNA vor der Spaltung durch DNAse geschützt hat. Auf demselben Gel werden daneben die verschiedenen Ansätze einer Sequenzierung nach Maxam-Gilbert aufgetragen, wodurch es ermöglicht wird, genau die Lage der Erkennungssequenz und ihre eventuelle Bindung durch das Antibiotikum festzustellen.

Als weiterer Orientierungsversuch muß noch überprüft werden, ob das verwendete Antibiotikum tatsächlich die Sternaktivitäten der Restriktionsendonuklease verringert. Dies geschieht dadurch, daß nebeneinander die zu spaltende DNA in Ansätzen jeweils mit bzw. ohne Antibiotikum inkubiert wird. Nach Auftrennung der entstandenen Fragmente in einem Agarosegel kann festgestellt werden, ob nach Antibiotikumzugabe die aufgrund der Sternaktivitäten aufscheinenden Banden im Gel verringert oder entfernt sind.

Es besteht jedoch auch die Möglichkeit, im erfindungsgemäßen Verfahren auf Antibiotika zurückzugreifen, deren bevorzugte Bindesequenz bereits bekannt ist. Derartige Antibiotika werden im erfindungsgemäßen Verfahren bevorzugt eingesetzt. Die folgende Tabelle 1 zeigt eine Auflistung der bevorzugten Antibiotika, deren Bindesequenz, sowie Literaturstellen, in denen diese Antibiotika näher beschrieben sind. Anhand der bekannten Bindesequenzen dieser Antibiotika kann leicht festgestellt werden, ob das Antibiotikum geeignet ist, Sternaktivitäten zu verringern oder/und ob zu erwarten ist, daß die spezifische Aktivität durch Bindung des Antibiotikums an die spezifische Erkennungssequenz der Restriktionsendonuklease beeinträchtigt wird.

## Tabelle 1

| Antibioticum | bevorzugte Bindesequenz | Literatur |
|---|---|---|
| Actinomycin D | GC | 1,2 |
| Distamycin | ATT oder AATT | 1,3 |
| Echinomycin | CG | 1,4 |
| Mithramycin | GC | 1 |
| Netropsin | $A/_T$ $A/_T$ $A/_T$ | 5,6 |

1. R.J. White & D.R. Phillips, Biochemistry 28 (1989), 6259-6269.
2. V.A. Aivasashvilli & R.S. Beabealashvilli, FEBS Lett. 160 (1983), 12-128.
3. M.L. Kopka, C. Yoon, D. Goodsell, O. Pjura & R.E. Dickerson, Proc. Natl. Acad. Sci. USA 82 - (1985), 1376-1380.
4. C.M.L. Low, H.R. Drew & M.J. Waring, Nucl. Acids Res. 12 (1984), 4865-4879.
5. H.M. Berman, S. Neidle, C. Zimmer & H. Thrum, Biochim. Biophys. Acta 561 124-131.
6. A.D.B. Malcolm & J.R. Moffat, Biochim. Biophys. Acta 655 (1981), 128-135.

Ob ein bestimmtes Restriktionsenzym Sternaktivitäten aufweist, ist leicht anhand der Spaltung von bekannten DNA-Sequenzen zu ermitteln, die eine bestimmte Anzahl von Erkennungssequenzen des Enzyms aufweisen. Treten nach erfolgter Spaltung dieser bekannten DNA-Sequenz durch das Enzym unerwartete Banden im Gel auf, so ist von einer Sternaktivität auszugehen. In der folgenden Tabelle 2 ist für einige Enzyme, für die es bekannt ist, daß sie Sternaktivitäten mehr oder weniger starken Ausmaßes besitzen, die Erkennungssequenz des Enzyms, sowie ein oder mehrere für die Verringerung der Sternaktivitäten verwendbares Antibiotikum ebenfalls mit seiner bevorzugten Bindesequenz angegeben.

3

## T a b e l l e  2

| Enzym | Erkennungsse-quenz des Enzyms | Antibioticum | bevorzugte Bindesequenz des Anti-bioticums |
|---|---|---|---|
| EcoRI | GAATTC | Actinomycin D | GC |
| SgrAI | C A CCGG T G<br>G · C | Netropsin | A/T A/T A/T |
| BamHI | GGATCC | Actinomycin D<br>Mithramycin | GC<br>GC |
| BfrI | CTTAAG | Actinomycin D | GC |
| EcoRV | GATATC | Actinomycin D<br>Mithramycin | GC<br>GC |
| HaeIII | GGCC | Netropsin<br>Distamycin | A/T A/T A/T<br>ATT oder AATT |
| HhaI | GCGC | Netropsin<br>Distamycin | A/T A/T A/T<br>ATT oder AATT |
| HindIII | AAGCTT | Echinomycin | CG |
| HpaI | GTTAAC | Actinomycin D | GC |
| PaeR7 | CTCGAG | Netropsin | A/T A/T A/T |
| PstI | CTGCAG | " | " |
| PvuII | CAGCTG | " | " |
| SalI | GTCGAC | " | " |
| SstI | GAGCTC | " | " |
| XbaI | TCTAGA | Echinomycin<br>Actinomycin D | CG<br>GC |

Die angegebenen Kombinationen von Restriktionsendonuklease und Antibiotikum sind bevorzugte Ausgestaltungen im erfindungsgemäßen Verfahren. Jedoch können auch andere Kombinationen von Enzym und Antibiotikum im erfindungsgemäßen Verfahren verwendet werden, solange durch die oben beschriebenen Orientierungsversuche festgestellt wurde, daß zwar die Sternaktivitäten verringert, jedoch die spezifische Aktivität der Restriktionsendonuklease nicht signifikant verändert wird.

Im erfindungsgemäßen Verfahren werden die Antibiotika vorzugsweise in einer Menge von 5 bis 200 $\mu$mol/l im Inkubationsansatz eingesetzt.

Das erfindungsgemäße Verfahren ermöglicht es auf einfache Weise, bei der Spaltung von Desoxyribonukleinsäuren durch Restriktionsendonukleasen des Typ II die Spezifität der Spaltung zu erhöhen, indem unspezifische Sternaktivitäten des Enzyms durch Bindung eines Antibiotikums an die Sternsequenzen des Enzyms auf der DNA vermindert werden. Geeignete Antibiotika können hierbei entweder aus den beispielshaft angegebenen bevorzugten Kombinationen von Enzym und Antibiotikum entnommen werden, oder aber durch einfache Vorversuche ermittelt werden. Es wird somit auf einfache und kostengünstige Weise ermöglicht, eine äußerst spezifische DNA-Spaltung durchzuführen, was für heutige gentechnologische Methoden äußerst wünschenswert ist, bisher jedoch nicht mit allen Enzymen erreicht werden konnte.

Die folgenden Beispiele sollen die Erfindung weiter erläutern.

## Beispiel 1

**Bestimmung der Aktivität und Nachweis von Sternaktivität für SgrAI**

a) Aktivität

Definition der Enzymeinheiten:

1 U SgrAI spaltet 1 $\mu$g Lambda-DNA innerhalb einer Stunde bei 37°C in 50 $\mu$l Endvolumen.

Zur Bestimmung der Aktivität werden steigende Mengen an Enzym (verschiedene Verdünnungen) mit 1 $\mu$g Lambda-DNA inkubiert.

Die Enzymmenge, die 1 $\mu$g Lambda-DNA in 1 Stunde bei 37°c vollständig spaltet, entspricht 1 U.

b) Aktivitätstest

Zu einer Mischung von 5 $\mu$l Inkubationspuffer (330 mmol/l Tris-Acetat, pH 7.9/37°C, 100 mmol/l Magnesiumacetat, 660 mmol/l Kaliumacetat, 5 mmol/l DTT) werden 39 $\mu$l (falls dem Reaktionsgemisch Netropsin zugesetzt wird 34 $\mu$l) Wasser und 5 $\mu$l Lambda-DNA (optische Dichte: 4 OD/ml) sowie 1 $\mu$l SgrAI (0,2 U/$\mu$l bis 15 U/$\mu$l) zugegeben. Die Lösung wird 1 Stunde bei 37°C inkubiert, auf Eis gekühlt und mit 10 $\mu$l eines Abstopp-Reagenses, bestehend aus 7 mol/l Harnstoff, 20 % (w/v) Saccharose, 60 mmol/l EDTA und 0.01 % (w/v) Bromphenolblau versetzt. Anschließend wird eine Trennung durch Elektrophorese in 0.8 %igen Agarosegelen für 2 bis 3 Stunden bei 100 Volt durchgeführt. Die erhaltenen Banden werden über Vergleich mit einem DNA-Längenstandard identifiziert.

c) Nachweis von Sternaktivität

Sternaktivität ist durch das Auftreten von Banden charakterisiert, die zusätzlich zum Bandenmuster des durch spezifische Spaltung entstandenen vollständigen Verdaus auftreten.

Die Herstellung der Inkubationsmischung und die Analyse der Spaltprodukte erfolgt analog zum Aktivitätstest. Die Inkubationsdauer beträgt jedoch 16 Stunden.

## Beispiel 2

Bestimmung der Aktivität und Nachweis der Sternaktivität für EcoRI

a) Aktivität

Definition der Enzymeinheiten

1 U EcoRI spaltet 1 $\mu$g Lambda-DNA innerhalb einer Stunde bei 37°C in 25 $\mu$l Endvolumen.

Zur Bestimmung der Aktivität werden steigende Mengen an Enzym (verschiedene Verdünnungen) mit 1 $\mu$g Lamda-DNA inkubiert. Die Enzymmenge, die 1 $\mu$g Lambda-DNA in 1 Stunde bei 37°C vollständig spaltet entspricht 1 U.

b) Aktivitätstest

Zu einer Mischung von 2.5 $\mu$l Inkubationspuffer (500 mmol/l Tris-HCl, pH 7.5/37°C, 1 mol/l NaCl, 100 mmol/l Magnesiumchlorid, 10 mmol/l DTE)werden 16.5 $\mu$l (wenn Aktivität in Gegenwart von Antibiotica bestimmt werden soll 14 $\mu$l) Wasser und 5 $\mu$l Lambda-DNA (optische Dichte: 4 OD/$\mu$l) sowie 1 bis 2 $\mu$l EcoRI-Lösung (0,3 bis 40 U/$\mu$l) zugegeben. Die Lösung wird eine Stunde bei 37°C inkubiert, auf Eis gekühlt und mit 10 $\mu$l eines Abstopp-Reagenzes, bestehend aus 7 mol/l Harnstoff, 20% (w/v) Saccharose, 60 mmol/l EDTA und 0.01% (w/v) Bromphenolblau versetzt. Anschließend wird eine Trennung durch Elektrophorese in 0,8 %-igen Agarosegelen für 2 bis 3 Stunden bei 100 Volt durchgeführt. Die erhaltenen Banden werden über Vergleich mit einem DNA-Längenstandard identifiziert.

c) Nachweis zur Sternaktivität

Sternaktivität ist durch das Auftreten von Banden charakterisiert, die zusätzlich zum Bandenmuster des durch spezifische Spaltung entstandenen vollständigen Verdaus auftreten.

Die Reaktionsmischung wird nach dem gleichen Verfahren hergestellt, wie für die Bestimmung der Aktivität beschrieben. Die Inkubationszeit beträgt jedoch 16 Stunden. Die Auftrennung der Spaltprodukte erfolgt ebenfalls wie bei der Aktivitätsbestimmung beschrieben.

**Beispiel 3**

**Nachweis, daß Netropsin außerhalb der Erkennungssequenz von SgrA1 bindet.**

Mit der "Footprinting"-Methode (White, R.J., & Phillips, D.R. (1989) Biochemistry 28, 6259-6269; Dynan, W.S., & Tjian, R. (1983) Gell 35, 79-87) wurde untersucht, ob Netropsin außerhalb der Erkennungssequenz für SgrAI bindet. Nach dieser Methode werden DNA-bindende Faktoren an DNA-Fragmente gebunden und diese dann so mit DNAse verdaut, daß maximal ein Schnitt pro Fragment entsteht. Bei der Auftrennung der Spaltprodukte im denaturierenden Polyacrylamidgel entsteht ein leiterartiges Muster. In diesen Leitern befinden sich dort Lücken, wo das Bindeprotein die DNA vor der Spaltung durch DNAse geschützt hat. Ein 191 Basenpaare langes Fragment, welches eine Spaltstelle für SgrAI enthält, wurde mit BamHI und SphI aus dem Plasmid pBR322 ausgeschnitten und an der BamHI-Spaltstelle mit $^{32}$Phosphor markiert. Jeweils 5 fmol dieses Fragmentes wurden mit 10 $\mu$mol/l und 20 $\mu$mol/l Netropsin inkubiert und mit DNAseI gespalten. Nach Auftrennung der Spaltprodukte im Polyacrylamid-Gel wurden mehrere geschützte Bereiche nachgewiesen. Die Erkennungssequenz für SgrAI war jedoch nicht durch Netropsin geschützt.

**Beispiel 4**

**Beispiele, in denen Antibiotica, welche an die Erkennungssequenz binden, die Sternaktivität nicht beeinflussen, oder außer der Sternaktivität auch die spezifische Aktivität vermindern.**

Die Bestimmung der Aktivität erfolgt für SgrAI wie im Beispiel 1, für EcoRI wie im Beispiel 2 beschrieben.

Die spezifische Aktivität von SgrAI, welches in der Erkennungssequenz eine Bindestelle für Actinomycin D hat, wird durch Actinomycin D (> 6 $\mu$mol/l) gehemmt.

Die spezifische Aktivität von EcoRI, welches in der Erkennungssequenz eine Bindestelle für Netropsin hat, wird durch Netropsin (200 $\mu$mol/l) gehemmt.

Die spezifische Aktivität und die Sternaktivität von Scal, welches in der Erkennungssequenz ebenfalls eine Bindungsstelle für Netropsin hat, wird durch Netropsin (<150 $\mu$mol/l) gehemmt.

**Aktivitätsbestimmung von Scal**

Definition der Enzymeinheiten: 1 U Scal spaltet ein 1 $\mu$g Lambda-DNA innerhalb einer Stunde bei 37 °C.

Zu einer Mischung von 25 $\mu$l Inkubationspuffer (500 mmol/l Tris-HCl, pH 7,5/37 °C, 100 mmol/l MgCl$_2$, 1 mol/l NaCl, 10 mmol/l DTE) werden 17,5 $\mu$l Wasser und 5 $\mu$l Lambda-DNA (optische Dichte: 4 OD/$\mu$l) sowie 1 $\mu$l Scal-Lösung (1 U/$\mu$l - 5 U/$\mu$l) zugegeben. Die Lösung wird 1 Stunde bei 37 °C inkubiert, auf Eis gekühlt und mit 5 $\mu$l eines Abstopp-Reagenses, bestehend aus 7 mol/l Harnstoff, 20 % (w/v) Saccharose, 60 mmol/l EDTA und 0,01 % (w/v) Bromphenolblau versetzt. Anschließend wird eine Trennung durch Elektrophorese in 1 %igen Agarosegelen für 2 - 3 Stunden bei 100 V durchgeführt. Die erhaltenen Banden werden durch Vergleich mit einem DNA-Längenstandard identifiziert.

**Beispiel 5**

**Nachweis der Bindung eines Antibioticums an die Erkennungssequenz oder außerhalb.**

Ein solches Verfahren ist die Footprinting Methode, die in Beispiel 3 beschrieben ist.

DNA-bindende Antibiotica werden in Konzentrationen von 5 bis 20 $\mu$mol/l an DNA-Fragmente gebunden. Diese DNA-Fragmente sollen die zu untersuchenden RE-Erkennungssequenzen enthalten. Die DNA-Fragmente werden an einem der beiden Stränge mit $^{32}$Phosphor endmarkiert. Jeweils 5 bis 10 fmol markiertes Fragment wird in einem Reaktionsvolumen von 50 $\mu$l mit 5 bis 20 $\mu$mol/l Antibioticum für 15 Minuten bei 0 °C inkubiert. Daraufhin wird der Reaktionsansatz für eine Minute bei Raumtemperatur inkubiert, 50 $\mu$l einer

Lösung aus MgCl$_2$ (10 mmol/l) und CaCl$_2$ (5 mmol/l) zugesetzt und wieder eine Minute bei Raumtemperatur inkubiert. 2 $\mu$l DNAse (0,125 ng/ml) werden zugegeben und eine Minute bei Raumtemperatur inkubiert. Die Reaktion wird durch Zugabe von 90 $\mu$l Stopplösung (20 mmol/l EDTA, 1 % SDS, 0,2 mmol/l NaCl, 250 $\mu$g/ml Hefe-RNA, pH 8,0) abgebrochen. Das Reaktionsgemisch wird phenolisiert, die DNA mit Ethanol gefällt und auf einem 6 %igen Polyacrylamidgel, welches 8 mol/l Harnstoff enthält, aufgetrennt. Der Nachweis der DNA erfolgt durch Autoradiographie. Die durch die Bindung des Antibioticums geschützten Bereiche zeichnen sich durch Lücken in der durch den limitierten DNAse-Verdau entstandenen Fragmentleitern ab. Die Zuordnung der Bindungsstellen zur Sequenz des DNA-Fragments geschieht durch parallel aufgetragene Sequenzleitern, die nach dem Verfahren von Maxam und Gilbert (Methods in Enzymology (1980) 65, 497-559) hergestellt werden.

**Beispiel 6**

Reduktion der Sternaktivität von SgrAI durch Netropsin:

Die Bestimmung der Aktivität und Sternaktivität von SgrAI in Gegenwart von Netropsin erfolgt wie in Beispiel 1 beschrieben. Dem Inkubationsgemisch werden 1,25 - 5 $\mu$l einer Lösung aus Netropsin (4 mmol/l Netropsin, 10 mmol/l Tris-HCl, 1 mmol/l EDTA pH 8,0) zugesetzt. Die Sternaktivität zeichnet sich durch das Auftreten von DNA-Fragmenten aus, die zusätzlich zu den durch die spezifische Aktivität erzeugten auftreten.

Bedingungen des Verdaus

1 $\mu$g Lambda-DNA, 0.1 bis 0.4 mmol/l Netropsin, 1 bis 20 U SgrAI, 33 mmol/l Tris-Acetat, 10 mmol/l Mg-Acetat, 66 mmol/l K-Acetat, 0.5 mmol/l DTT, pH 7.9 bei 37° C. Volumen der Reaktionsmischung 50 $\mu$l. Inkubation für 16 Stunden bei 37° C.

Durch Spaltung von Lambda-DNA mit SgrAI an den Erkennungssequenzen CA/GCCGGC/TG enstehen sieben Fragmente mit
16 676
14 850
7 066
4 198
2 775
1 616 und
1 321 Basenpaaren.

Diese werden durch Elektrophorese aufgetrennt und durch Anfärben mit Ethidiumbromid im UV-Licht sichtbar gemacht.

Steigende Mengen SgrAI wurden mit jeweils 1 $\mu$g Lambda-DNA für 16 Stunden bei 37° C inkubiert.

In Abwesenheit von Netropsin ist bei Enzymmengen zwischen 1 bis 5 U das für den vollständigen Verdau charakteristische Muster (sieben Banden) zu beobachten. Mit 7 U ist das Fragment mit 1 616 bp zum Teil degradiert. Mit 10 U treten zusätzliche Banden schwacher Intensität auf, die Fragmenten mit ca. 13 000 bp und ca. 10 500 bp zugeordnet werden können. Mit 12 U SgrAI sind die Fragmente mit ca. 13 000bp und 10 500 bp deutlicher sichtbar, es sind weitere Fragmente mit ca 3 800, 3 700 und 2 000 Basenpaaren entstanden und das Fragment mit 1 616 bp fehlt vollständig. Mit 16 U SgrAI werden weitere zusätzliche Fragmente gebildet, während die durch spezifischen Verdau entstandenen Fragmente stärker degradiert sind.

Bei Zusatz von 0.2 mmol/l Netropsin ist bis 16 U SgrAI keine durch Sternaktivität erzeugte Bande zu beobachten, das 1616 bp-Fragment ist ab 12 U SgrAI leicht degradiert. Mit 0.4 mmol/l Netropsin ist auch bis 16 U kein zusätzliches Fragment sichtbar, das durch Sternaktivität entstanden sein könnte, das Fragment mit 1616 bp ist nur geringfügig degradiert.

Die Sternaktivität ist durch Netropsin um das 2- bis 3-fache reduzierbar.

**Beispiel 7**

Reduktion der Sternaktivität von EcoRI durch Actinomycin D:

Die Bestimmung der Aktivität und Sternaktivität von EcoRI in Gegenwart von Actinomycin D erfolgt analog nach dem in Beispiel 2 beschriebenen Verfahren. Dem Inkubationsansatz werden jedoch 1,25 - 2,5 $\mu$l einer Lösung aus Actinomycin D (200 $\mu$mol/l Actinomycin D, 10 mmol/l Tris-HCl, 1 mmol/l EDTA, pH 8,0) in TE zugesetzt.

Spezifische Spaltung von Lambda-DNA an der vollständigen Erkennungssequenz von EcoRI führt zu Fragmenten mit

21 226

7 421

5 804

5 643

4 878 und

3 530 Basenpaaren.

Bedingungen des Verdaus

1 μg Lambda-DNA, 10 - 20 μmol/l Actinomycin D, 1 bis 60 U EcoRI, 50 mmol/l Tris-HCl, 10 mmol/l MgCl$_2$, 100 mmol/l NaCl, 1 mmol/l DTE, pH 7.5 bei 37° C. Volumen der Reaktionsmischung 25 μl. Inkubation für 16 h bei 37° C.

Wenn 1 μg Lambda-DNA mit steigenden Mengen EcoRI für 16 Stunden inkubiert wird, werden folgende Beobachtungen gemacht:

1 bis 10 U EcoRI ergeben das vollständige Verdau-Muster der Lambda-DNA. Mit 20 U EcoRI treten zusätzliche Banden auf, die Fragmenten der ungefähren Größe von 11 500, 8 800, 4500, 2 800 und 2000 Basenpaaren zugeordnet werden können. Die durch spezifische Spaltung entstandenen Fragmente sind teilweise degradiert, wie aus der relativen Intensität ersehen werden kann. Mit 30 U EcoRI werden die durch für spezifische Spaltung charakteristischen Fragmente weiter degradiert, die durch unspezifische Spaltung entstandenen Fragmente werden zahlreicher und sind in größerer Menge vorhanden.

Wird dem Reaktionsgemisch jedoch Actinomycin D (10 μmol/l) zugesetzt, sind die Spaltmuster, die durch 10 U, 20 U und 30 U EcoRI entstehen identisch, Spaltung tritt nur an der spezifischen Erkennungssequenz von EcoRI auf. Erst mit 40 U EcoRI entstehen zusätzliche Banden. Die Sternaktivität ist um das 3-fache reduziert.

## Patentansprüche

1. Verfahren zur Verminderung von unspezifischen Sternaktivitäten bei der spezifischen Spaltung von Desoxyribonukleinsäuren durch Inkubation mit einer Restriktionsendonuklease in einem geeigneten Puffer, **dadurch gekennzeichnet,** daß man dem Inkubationsansatz ein Antibiotikum zusetzt, welches an oder nahe der Sternsequenzen des Enzyms, jedoch nicht innerhalb der spezifischen Erkennungssequenz der Restriktionsendonuklease an die DNA bindet.

2. Verfahren nach Anspruch 1, **dadurch gekennzeichnet,** daß man zur Untersuchung, ob ein Antibiotikum an die spezifische Erkennungssequenz der Restriktionsendonuklease bindet, einen sogenannten "footprint assay" durchführt und die Bindung des Antibiotikums an Sternsequenzen durch Vergleichsversuche zur DNA-Spaltung mit der Endonuklease mit und ohne Antibiotikum im Inkubationsansatz feststellt.

3. Verfahren nach Anspruch 1 oder 2, **dadurch gekennzeichnet,** daß man abhängig von der verwendeten Restriktionsendonuklease als Antibiotikum Actinomycin D, Distamycin, Echinomycin, Mithramycin oder Netropsin verwendet.

4. Verfahren nach Anspruch 1, **dadurch gekennzeichnet,** daß man Actinomycin D dem Inkubationsansatz bei Spaltung mit den Endonukleasen BamHI, Bfrl, EcoRV, Hpal und Xbal zusetzt.

5. Verfahren nach Anspruch 1, **dadurch gekennzeichnet,** daß man Mithramycin dem Inkubationsansatz bei Spaltung mit BamHI oder EcoRV zusetzt.

6. Verfahren nach Anspruch 1, **dadurch gekennzeichnet,** daß man Netropsin dem Inkubationsansatz bei Spaltung mit HaeIII, Hhal, PaeR7, Pstl, Pvull, Sall oder Sstl zusetzt.

7. Verfahren nach Anspruch 1, **dadurch gekennzeichnet,** daß man Distamycin dem Inkubationsansatz bei Spaltung mit HaeIII oder Hhal zusetzt.

8. Verfahren nach Anspruch 1, **dadurch gekennzeichnet,** daß man Echinomycin dem Inkubationsansatz bei Spaltung mit HindII oder XbaI zusetzt.

9. Verfahren nach einem der vorhergehenden Ansprüche, **dadurch gekennzeichnet,** daß man das Antibiotikum in einer Menge von 5 bis 200 $\mu$mol/l dem Inkubationsansatz zusetzt.